# EUROPÄISCHE PATENTSCHRIFT

(11) **EP 0 889 035 B1**
(45) Veröffentlichungstag und Bekanntmachung des Hinweises auf die Patenterteilung: **13.10.2004**
(21) Anmeldenummer: 98111938.1
(22) Anmeldetag: 27.06.1998
(51) Int. Cl.: C07D 215/04, B01D 9/00, B01D 3/14

(54) **Verfahren zur Reinigung von Chinaldin**
Process for the purification of quinaldine
Procédé pour la purification de quinaldine

(30) Priorität: 05.07.1997 DE 19728835
(43) Veröffentlichungstag der Anmeldung: 07.01.1999
(73) Patentinhaber: RÜTGERS Chemicals AG, 44579 Castrop-Rauxel (DE)
(72) Erfinder: Talbiersky, Jörg, Dr., 46282 Dorsten (DE); Fuhrmann, Edgar, Dr., 44579 Castrop-Rauxel (DE); Janneck, Heinz-Günter, 45711 Datteln (DE)
(74) Vertreter: COHAUSZ & FLORACK

(56) Entgegenhaltungen:
- GB-A- 736 589
- US-A- 2 432 064
- CHEMICAL ABSTRACTS, vol. 106, no. 1, 5. Januar 1987 Columbus, Ohio, US; abstract no. 4899e, TODA F.: "Separation and purification of quinaldine" Seite 461; Spalte 1; XP002081150 & JP 61 093165 A (NIPPON STEEL CHEMICAL CO., LTD.) 12. Mai 1986

## Beschreibung

Die Erfindung betrifft ein Verfahren zur Reinigung von Chinaldin ausgehend von einer Chinaldinfraktion aus der Steinkohlenteeraufarbeitung, in dem man Chinaldin mit einkernigen Aromaten unter Adduktbildung auskristallisieren läßt und das Chinaldin/Aromaten-Addukt trennt.

Der die Chinaldinfraktion enthaltene Methylnaphthalinstrom aus der Steinkohlenteeraufarbeitung enthält verschiedene Chinolinbasen, deren Siedepunkte sehr nah beieinander liegen. So besitzt 8-Methylchinolin einen Siedepunkt von 247,75°C, 2-Methylchinolin (Chinaldin) einen Siedepunkt von 247,6°C und Isochinolin einen Siedepunkt von 243°C. In dieser Fraktion ist Chinaldin in einer Konzentration von etwa 60 % enthalten, eine destillative Anreicherung auf Qualitäten größer als 90 % ist wegen der Siedebegleiter, insbesondere 8-Methylchinolin, nicht möglich.

Aus der US 2 432 064 ist die Adduktbildung von Rohchinaldin mit einer einkernigen Teersäure bekannt, die Chinaldinqualitäten mit einer Reinheit von 95 bis 98% ermöglichen soll. Es hat sich jedoch herausgestellt, daß dieses Verfahren nicht in reproduzierbarer Weise nachgearbeitet werden kann.

Die GB 736 589 beschreibt die Gewinnung von Chinaldin durch Isolierung eines Chinaldin/Harnstoff-Addukts und Spaltung des Addukts mit Wasser. Die Adduktbildung erfolgt durch Reaktion einer Chinaldinfraktion/Toluol-Lösung mit einer wässrigen Harnstofflösung bei 80°C und Abkühlen des Reaktionsgemischs, wobei der Chinaldin/Harnstoff-Komplex in der Kälte ausfällt. Nach Abtrennung des Präzipitats und Erhitzen desselben in einem Gemisch aus Toluol und Wasser geht Chinaldin in die Toluolphase über und läßt sich somit von der wässrigen Harnstofflösung trennen.

Der Erfindung liegt deshalb die Aufgabe zugrunde, ein reproduzierbares Verfahren bereitzustellen, das die Gewinnung von Chinaldin in noch größerer Reinheit als das bekannte Verfahren und reproduzierbarer Weise ermöglicht.

Gelöst wird diese Aufgabe durch ein Verfahren zur Reinigung von Chinaldin, in dem man einer Chinaldinfraktion aus der Steinkohlenteeraufarbeitung einen einkernigen Aromaten vom Typ Phenol, Kresol und Xylenol zusetzt, diesen unter Adduktbildung mit Chinaldin auskristallisieren läßt und das Chinaldin/einkerniger Aromat-Addukt anschließend trennt, wobei man der Chinaldinfraktion vor Zugabe des einkernigen Aromaten 5 bis 20 Gew.% eines Aromatengemischs (Arsol) oder Toluol, bezogen auf die Masse der Chinaldinfraktion, hinzufügt.

Als Rohstoffquelle für die Gewinnung von hochreinem Chinaldin kann eine Chinaldinfraktion aus der Steinkohlenteeraufarbeitung eingesetzt werden, die destillativ erhalten wird und Chinaldin in einer Menge von etwa 60 Gew.% enthält.

Gemäß einer bevorzugten Ausführungsform werden der Chinaldin enthaltenden Fraktion 15 bis 20 Gew.% Toluol oder Arsolgemisch und gemäß einer besonders bevorzugten Ausführungsform etwa 18 Gew.%, bezogen auf das Gewicht der Chinaldin enthaltenden Fraktion, zugesetzt.

Unter Arsolaromatengemisch werden erfindungsgemäß Aromatengemische verstanden, die Toluol, Benzol, Xylole, Ethylbenzole, Propylbenzole, Indan, Inden, hochsiedende Verbindungen und weitere nicht identifizierte Verbindungen enthalten können. Diese Aromatengemische fallen beispielsweise als Sumpfprodukt in einer Xylol-Hauptkolonne bei der Rohbenzol-Aufarbeitung als das zwischen 165 und 180 °C siedende Arsol I und das zwischen 180 und 200 °C siedende Arsol II an.

Nach der erfindungsgemäßen Behandlung mit Toluol oder dem Arsol-Aromatengemisch erfolgt zur Adduktbildung die Zugabe des einkernigen Aromaten vom Typ Phenol, Kresol und Xylenol. Es können o-Kresol, m-Kresol und p-Kresol sowie 1,3,4-Xylenol und 1,2,4-Xylenol eingesetzt werden. Besonders bevorzugt ist o-Kresol. Dieses ist gemäß einer weiteren bevorzugten Ausführungsform ein teerstämmiges o-Kresol.

Bekanntlich kann teerstämmiges o-Kresol Basengehalte von mehr als 1% aufweisen. Es ist dennoch möglich, solche Qualitäten ohne Nachteile zu verwenden. Ferner entsteht in diesem Fall bei Anwendung des beschriebenen Verfahrens überraschenderweise ein hochprozentiges, praktisch basenfreies o-Kresol. Diese Möglichkeit zur Raffination von basenhaltigem o-Kresol erhöht gemäß einer bevorzugten Ausführungsform der Erfindung das Wirtschaftlichkeitspotential des erfindungsgemäßen Verfahrens. Die Erfindung berifft somit auch ein Verfahren zur Entfernung von Basen aus teerstämmigem o-Kresol.

Nach Adduktbildung wird das Gemisch auf eine Temperatur von 0 bis 25°C, vorzugsweise 10 bis 25°C abgekühlt, wobei das Addukt spontan aus dem Gemisch ausfällt. Vorzugsweise erfolgt die Fällung des Gemischs in einem Kristaller gemäß der EP 148 511 A2, in dem die Kühlflächen in einem Winkel von mehr als 0° zur Senkrechten oder Waagerechten angeordnet sind. Das flüssige auszukristallisierende Gemisch befindet sich dabei in Ruhe oder bewegt sich maximal mit einer Geschwindigkeit, bei der gerade noch keine Kristallablösung von den Kühlflächen und kein Abbrechen der Kristalle aus der Kristallschicht stattfindet.

Der Einsatz eines solchen Schrägflächenkristallers ermöglicht eine kurze Kristallisationsstrecke und bietet den Vorteil einer einstufigen Kristallisation.

Das Chinaldin/o-Kresol-Addukt hat mit etwa 65°C einen recht hohen Schmelzpunkt. Die Adduktbildung erfolgt mit einer hohen Selektivität von 99,3% Adduktreinheit, bezüglich Chinaldin/o-Kresol. Das Addukt ist thermisch und damit auch destillativ in seine Grundbestandteile aufspaltbar.

Das in einer Laborkolonne erzeugte Chinaldin hat einen durchschnittlichen Gehalt von 98,7%, wobei 50% der Erzeugung eine Reinheit von größer als 99% aufweist.

Ferner kann auch das o-Kresol mit Ausnahme der Destillationsverluste aus der Mutterlauge der Adduktbildung als Leichtsieder zurückgewonnen werden. Das o-Kresol aus der Adduktdestillation hat einen durchschnittlichen Gehalt von 99,5%, in der Spitze einen Gehalt von 99,9% und kann daher für die Adduktbildung rezykliert werden oder als hochreine Ware in den Verkauf gelangen.

Das erfindungsgemäße Verfahren wird anhand des folgenden Beispiels näher erläutert.

### Beispiel

### Herstellung von Chinaldin 99% nach dem o-Kresol-Adduktverfahren

2.520 g Chinadinfraktion 64% (1) werden bei 20°C mit 441 g Arsol-Aromatengemisch (3) in einem 10-1-Becherglas gemischt. Anschließend werden 2.016 g teerstämmiges o-Kresol (2) mit der oben genannten Lösung vereinigt. Die Lösung erwärmt sich durch die freiwerdende Reaktionswärme auf etwa 70°C. Das gesamte Gemisch wird durch Abkühlen auf 20°C in einem Laborschrägflächenkristaller zur Kristallisation gebracht. Die Mutterlauge wird durch Abtropfen vom Kristallgut abgetrennt und das Kristallgut mit 441 g Arsol-Aromatengemisch gewaschen. Das gewaschene Kristallgut (4), 1650 g, besteht weitestgehend aus o-Kresol, Chinaldin und Arsol-Aromatengemisch. Dieses Kristallgut wird aufgeschmolzen und durch fraktionierende Rektifikation in einer 2 m Laborkolonne (Drahtgewebefüllkörper) aufgetrennt. Man erhält so ein basenarmes o-Kresol (> 500 ppm Basen) und ein hochreines Chinaldin (5), 790 g mit einem Chinaldingehalt von etwa 99,4%.

Die Verfahrensführung wird anhand des folgenden Diagramms erläutert, die Zusammensetzung der Produkte und zugegebenen Stoffe oder Stoffgemische ist anschließend dargestellt. 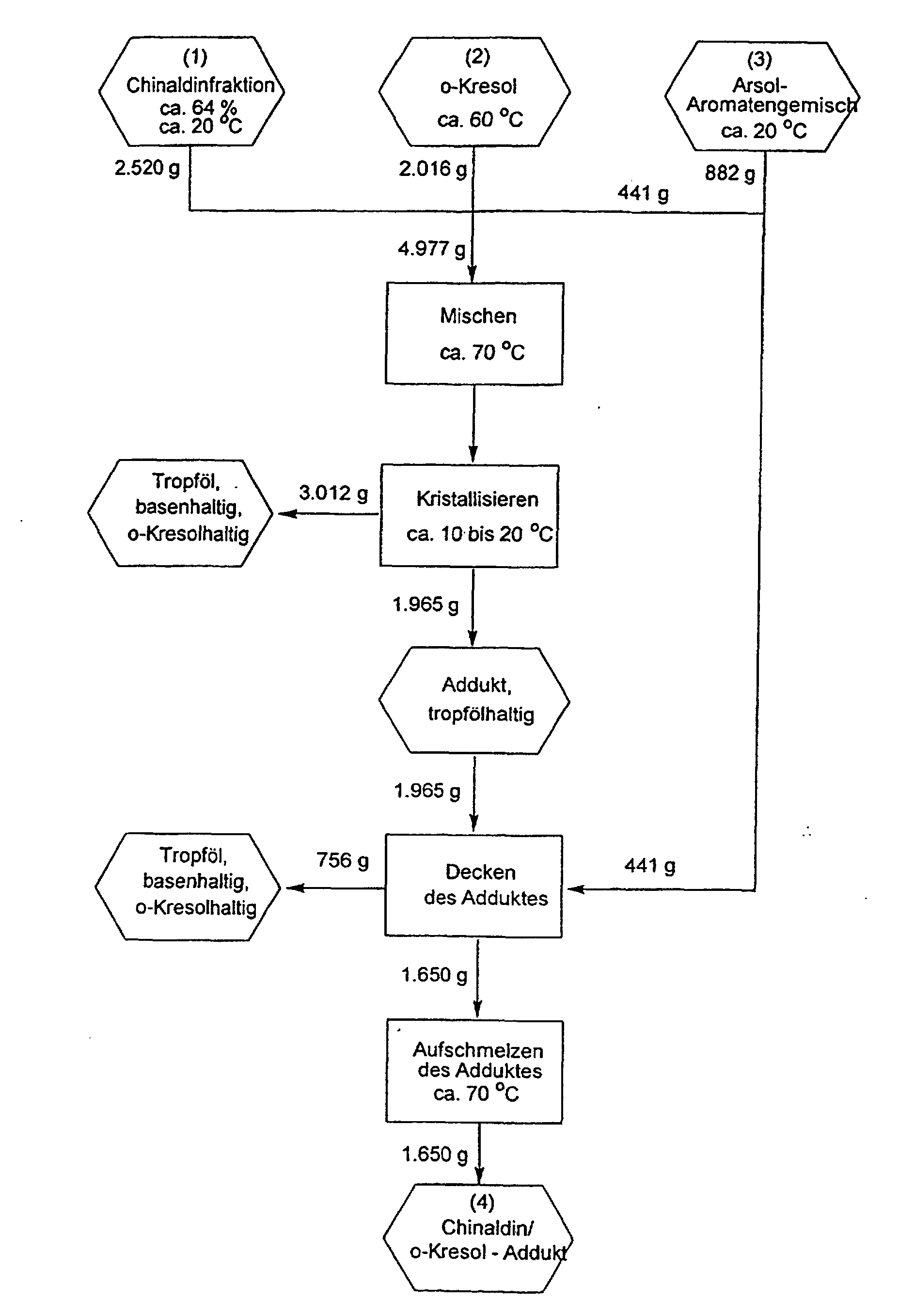 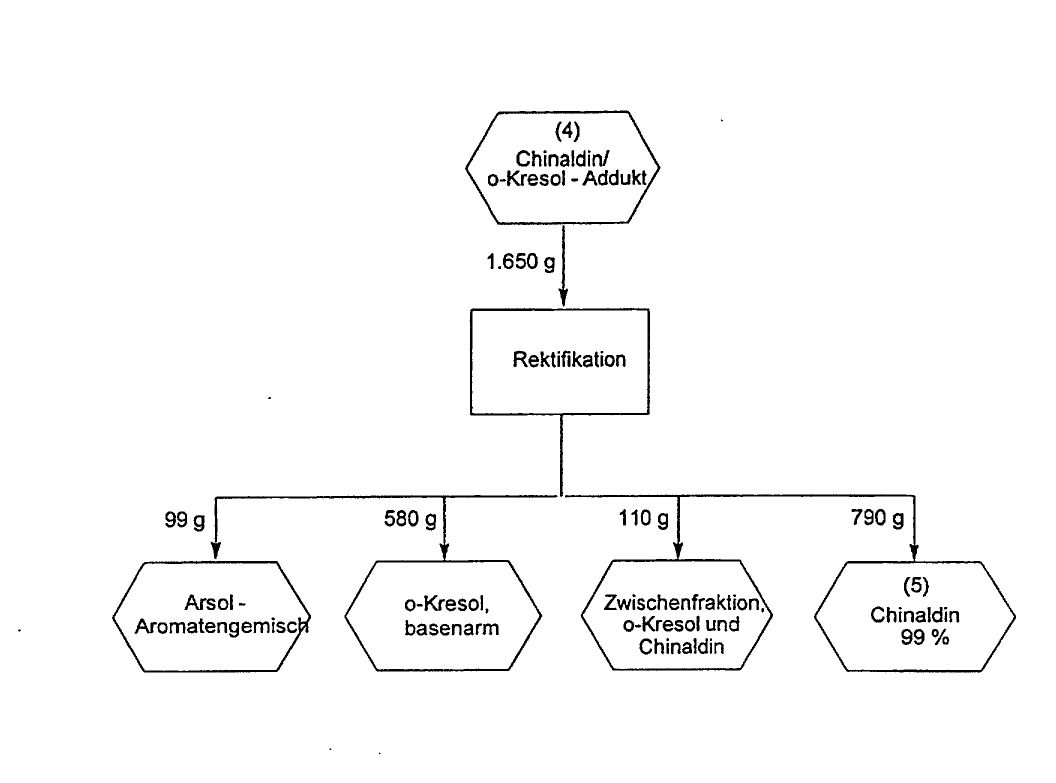
(1) Chinaldinfraktion (Massen%): Leichtsieder 2, Chinolin <2, Isochinolin 10-20, Chinaldin 60-70, 8-Methylchinolin 5-10, 3-Methylisochinolin 3-8, Lepidine <5, Hochsieder <10,
(2) o-Kresol-Zusammensetzung (Massen%): Phenol 0,5, o-Kresol 98,2, m/p-Kresol 0,5, Basen 0.8.
(3) Arsol-Aromatengemisch (Massen%): Benzol 6, Toluol 8, Xylole 13, Ethylbenzole 3, C3-Benzole 34, Indan 14, Inden 6, nicht identifizierte Verbindungen 14, Hochsieder 2.
(4) Chinaldin/o-Kresol-Addukt (Massen%): BTX-Aromaten 5,4, o-Kresol 40,2, Isochinolin 0,2, unbekannte Verbindungen 0,2 Chinaldin 53,4, 8-Methylchinolin 0,3, 3-Methylisochinolin 0,2, Lepidin 0,1.
(5) Chinaldin 99% (Massen%): Isochinolin 0,05, unbekannte Verbindungen 0,1, Chinaldin 99,4, 8-Methylchinolin 0,4, 3-Methylisochinolin 0,05.

## Patentansprüche

1. Verfahren zur Reinigung von Chinaldin aus der Steinkohlenteeraufarbeitung, in dem man der Chinaldinfraktion einen einkernigen Aromaten vom Typ Phenol, Kresol und Xylenol zusetzt, diesen unter Adduktbildung mit Chinaldin auskristallisieren läßt und das Chinaldin/einkerniger Aromat-Addukt anschließend trennt, **dadurch gekennzeichnet, daß** man der Chinaldinfraktion vor Zugabe des einkernigen Aromaten 5 bis 20 Gew.% eines Aromatengemischs oder Toluol, bezogen auf die Masse der Chinaldinfraktion, hinzufügt.

2. Verfahren nach Anspruch 1, **dadurch gekennzeichnet, daß** man das Addukt aus Chinaldin und einkernigem Aromaten in einem Kristaller auskristallisieren läßt, in dem die Kühlflächen in einem Winkel von mehr als 0° zur Senkrechten oder Waagerechten angeordnet sind.

3. Verfahren nach Anspruch 1, **dadurch gekennzeichnet, daß** man ein Basen enthaltendes o-Kresol aus der Steinkohlenteerverarbeitung zur Adduktbildung mit Chinaldin einsetzt.

## Claims

1. A method of purifying quinaldine processed from coal tar, in which a phenol, cresol and xylenol-type mononuclear aromatic compound is added to the quinaldine fraction and in which the aromatic compound is crystallized out forming an adduct with quinaldine followed by separating the quinaldine/mononuclear aromatic compound adduct, wherein before the mononuclear aromatic compound is added to the quinaldine fraction, 5% to 20% by weight of an aromatic mixture or toluene, based on the mass of the quinaldine fraction, is added to the quinaldine fraction.

2. The method of claim 1, wherein the adduct formed from quinaldine and mononuclear aromatic compounds is crystallized out in a crystallizer in which the cool surfaces are arranged at an angle of more than 0° to the vertical or horizontal.

3. The method of claim 1, wherein an o-Cresol prepared from coal tar and that contains bases is used to form the adduct with quinaldine.

## Revendications

1. Procédé de purification de quinaldine provenant du traitement de goudron de houille, dans lequel on ajoute à la fraction quinaldine, un aromatique monocyclique du type phénol, crésol et xylénol, celui-ci est cristallisé avec formation d'adduit avec la quinaldine et l'adduit quinaldine/aromatique monocyclique est ensuite séparé, **caractérisé en ce que** l'on ajoute à la fraction quinaldine, avant addition de l'aromatique monocyclique, 5 à 20% en poids d'un mélange d'aromatiques ou de toluène, sur base de la masse de la fraction quinaldine.

2. Procédé selon la revendication 1, **caractérisé en ce que** l'on fait cristalliser l'adduit de quinaldine et de l'aromatique monocyclique dans un cristallisoir, dans lequel la surface froide est disposée avec un angle de plus de 0° par rapport à la verticale ou horizontalement.

3. Procédé selon la revendication 1, **caractérisé en ce que** l'on met en oeuvre un o-crésol contenant des bases, provenant du traitement du goudron de houille, pour la formation de l'adduit avec la quinaldine.
